# EUROPÄISCHE PATENTSCHRIFT

(11) **EP 4 352 372 B1**
(45) Veröffentlichungstag und Bekanntmachung des Hinweises auf die Patenterteilung: **06.08.2025**
(21) Anmeldenummer: 22731216.2
(22) Anmeldetag: 01.06.2022
(51) Int. Cl.: F16C 1/26, A61F 5/01, A61H 1/02

(54) **MEDIZINISCHES HILFSMITTEL ODER SPORTHILFSMITTEL, UMFASSEND EINEN BOWDENZUG**
MEDICAL AID OR SPORTS AID COMPRISING A BOWDEN CABLE
ACCESSOIRE MÉDICAL OU ACCESSOIRE DE SPORT COMPRENANT UN CÂBLE BOWDEN

(30) Priorität: 07.06.2021 DE 102021205698
(43) Veröffentlichungstag der Anmeldung: 17.04.2024
(73) Patentinhaber: Bauerfeind AG, 07937 Zeulenroda-Triebes (DE)
(72) Erfinder: STIER, Gerald, 07937 Zeulenroda-Triebes (DE); HEBENSTREIT, Sandro, 07937 Zeulenroda-Triebes (DE); BAUERFEIND, Hans B., 07937 Zeulenroda-Triebes (DE)
(74) Vertreter: Wohlfahrt, Jan Günther
(86) Internationale Anmeldenummer: PCT/EP2022/064865
(87) Internationale Veröffentlichungsnummer: WO 2022/258448

(56) Entgegenhaltungen:
- WO-A1-2014/109799
- DE-A1- 102009 003 999
- DE-C- 177 927
- US-A- 2 092 898
- US-A- 2 509 445

## Beschreibung

Die vorliegende Erfindung betrifft ein medizinisches Hilfsmittel oder Sporthilfsmittel, umfassend einen Bowdenzug, wobei der Bowdenzug eine Seele umfasst, wobei die Seele zumindest in einem Teilbereich in einer Ummantelung verläuft, wobei die Ummantelung zumindest in einem Teilbereich dehnbar ist und die Verwendung eines Bowdenzugs, aufweisend eine Seele zum Spannen eines medizinischen Hilfsmittels oder eines Sporthilfsmittels, wobei die Seele zumindest teilweise in einer Ummantelung verschiebbar verläuft und die Ummantelung dehnbar ist.

Ein Bowdenzug ist ein bewegliches Maschinenelement zur Übertragung einer mechanischen Bewegung sowie von Druck- und Zugkräften mittels einer biegsamen Kombination aus einem Seil, insbesondere Drahtseil und einer in Verlaufsrichtung druckfesten Hülle beziehungsweise Ummantelung. Ein Bowdenzug besteht meist aus einem Zugseil, auch Seele genannt, beispielsweise einem Stahldraht oder Drahtseil, das in einer flexiblen aber in Zurichtung druckfesten Ummantelung, auch Hülle genannt, verschiebbar verlegt wird. Die Ummantelung dient der Führung des Seils sowie als Gegenlager zur Abstützung der zu übertragenden Zugkräfte. Ein Bowdenzug kann dadurch Zugkräfte entlang einer beliebig gekrümmten Bahn übertragen, solange ein gewisser Radius nicht unterschritten wird. Bowdenzüge werden in den verschiedensten technischen Gebieten eingesetzt, insbesondere aber im Fahrzeugbau. In der Medizintechnik wird die Verwendung von Bowdenzügen bei Gelenkorthesen in der EP 1 186 279 A1 und der DE 10 2009 003 999 A1 beschrieben. DE 177 927 C beschreibt ebenfalls einen gattungsgemäßen Bowdenzug nach dem Oberbegriff des Anspruchs 1.

Bei medizinischen Hilfsmitteln oder Sporthilfsmitteln werden anstelle von Bowdenzügen meist einfach Seilzüge verwendet, bei denen Seile oder Schnüre beispielsweise durch Aufrollen gespannt werden. Dabei können die Seile oder Schnüre frei verlaufen oder sie sind in Kaschierungen oder gestrickte Kanäle geführt.

Nachteilig ist bei solchen Seilen, dass sie zum einen bei der Bewegung auf dem medizinischen Hilfsmittel oder Sporthilfsmittel, beispielsweise auf einem Gestrick, reiben und somit das Gestrick auf Dauer beschädigen können, und darüber hinaus in die Haut des Trägers des Hilfsmittels einschneiden können.

Das der vorliegenden Erfindung zugrunde liegende technische Problem ist die Bereitstellung von Spannmitteln bei medizinischen Hilfsmitteln oder Sporthilfsmitteln, die die Probleme aus dem Stand der Technik vermeiden. Insbesondere ist das technische Problem der vorliegenden Erfindung die Bereitstellung von Alternativen zu Seilzügen bei medizinischen Hilfsmitteln oder Sporthilfsmitteln, insbesondere bei der Verwendung als Spannmittel, die beim Bewegen des Seilzugs, insbesondere des Spannmittels insbesondere in Umlenkbereichen und anderen Bereichen, bei denen Spannmittel häufig zum Klemmen neigen, weniger oder keine Reibung verursachen.

Die vorliegende Erfindung löst das ihr zugrunde liegende technische Problem durch ein medizinisches Hilfsmittel oder Sporthilfsmittel, umfassend einen Bowdenzug, wobei der Bowdenzug eine Seele umfasst, wobei die Seele zumindest in einem Teilbereich in einer Ummantelung verläuft, wobei die Ummantelung zumindest in einem Teilbereich dehnbar ist.

Es zeigte sich überraschender Weise, dass sich bei medizinischen Hilfsmitteln, insbesondere orthopädischen Hilfsmitteln, und Sporthilfsmitteln Bowdenzüge in vorteilhafter Weise anstelle von Seilzügen einsetzen lassen, insbesondere als Spannelemente einsetzen lassen, wenn bei den Bowdenzügen die Ummantelung, also die Hülle, zumindest in einem Teilbereich dehnbar ist. Bei herkömmlichen Bowdenzügen und einer herkömmlichen Verwendung dieser muss die Hülle nicht dehnbar sein, sondern nur biegsam, da die Seele nur in der Hülle verschoben wird und somit keine Verkürzung oder Verlängerung der Ummantelung nötig ist. Dies ist jedoch bei der Verwendung der Seele als Spannelement in einem medizinischen Hilfsmittel oder Sporthilfsmittel nötig, sodass hier ein herkömmlicher Bowdenzug nicht verwendbar wäre. Die Ausgestaltung mindestens eines Teilbereichs der Ummantelung als dehnbare Ummantelung führt nun in vorteilhafter Weise dazu, dass der Bowdenzug insgesamt in der Länge flexibel ist und so auch als Spannelement bei einem medizinischen Hilfsmittel oder Sporthilfsmittel verwendet werden kann.

Dem Fachmann sind verschiedene Möglichkeiten bekannt, wie er zumindest einen Teilbereich der Ummantelung oder die Ummantelung an sich dehnbar ausgestalten kann. Insbesondere kann die Ummantelung durch Schlitze dehnbar gemacht werden, beispielsweise durch einen spiralförmig geführten Schlitz. Alternativ kann die Ummantelung des Bowdenzugs auch, zumindest in Teilbereichen, Ziehharmonika-förmig ausgestaltet sein, oder aus einem dehnbaren Kunststoff oder Gummi gefertigt sein.

In einer bevorzugten Ausführungsform ist die Ummantelung aus Kunststoff.

In einer bevorzugten Ausführungsform weist die Ummantelung mindestens einen Schlitz auf.

In einer bevorzugten Ausführungsform ist die Ummantelung zumindest in einem Teilbereich spiralförmig ausgebildet.

Die Ummantelung kann durchgehend oder nur in Teilbereichen geschlitzt sein. Dabei kann die Schlitzung entweder ringförmig, also insbesondere spiralförmig, oder mäanderförmig ausgebildet sein. Die Schlitzung der Ummantelung kann auch nicht ringförmig ausgestaltet sein. So können die Schlitze beispielsweise versetzt, parallel oder schrägstehend ausgestaltet sein.

Eine Ummantelung mit mindestens einem Schlitz, insbesondere einem spiralförmigen Schlitz oder die Ausgestaltung einer an sich spiralförmigen Ummantelung, hat den Vorteil, dass mindestens ein Teilbereich der Ummantelung, aber auch die gesamte Ummantelung dehnbar ausgestaltet sein kann und gleichzeitig die Seele in der Ummantelung dennoch reibungsarm verschoben werden kann. Dadurch kann sich in vorteilhafter Weise der anfallende Reibungskoeffizient auf Seele und Ummantelung aufteilen.

In einer bevorzugten Ausführungsform ist die Ummantelung zumindest in einem Teilbereich elastisch.

In einer bevorzugten Ausführungsform weist die Ummantelung einen runden Querschnitt aufweist oder einen stadionförmigen Querschnitt auf. Der Querschnitt der Ummantelung, insbesondere der Querschnitt der Außenfläche der Ummantelung kann auch anders ausgestaltet sein, beispielsweise eckig, insbesondere mehreckig, oder oval.

Die Ummantelung kann also beispielsweise einen runden Querschnitt aufweisen, wie er für Bowdenzüge aus dem Stand der Technik üblich ist. Alternativ kann die Ummantelung, zumindest in Teilbereichen, auch einen stadionförmigen Querschnitt aufweisen. Ein stadionförmiger Querschnitt ist ein Querschnitt, bei dem zwei parallel verlaufende Geraden durch zwei Bögen miteinander verbunden sind. Die Ummantelung mit einem stadionförmigen Querschnitt ist dann insbesondere als innen hohles Flachband ausgestaltet. Damit kann die Ummantelung in vorteilhafter Weise mit einer der beiden flachen, geraden Flächen auf dem medizinischen Hilfsmittel oder Sporthilfsmittel aufliegen. Dadurch schneidet das Flachband, insbesondere wenn der Bowdenzug als Spannelement ausgebildet ist, nicht so sehr in die unter dem Bowdenzug liegende Haut des Benutzers ein.

Die Seele kann als herkömmliche Seele ausgestaltet sein, beispielsweise als Draht oder Seil. Die Seele kann aus Metall, Kunststoff oder anderen Materialeien ausgestaltet sein. Die Seele kann aber auch als Flachband ausgestaltet sein, beispielsweise wenn sie in einer als Flachband ausgestalteten Ummantelung verläuft.

In einer bevorzugten Ausführungsform ist der Bowdenzug als Spannelement ausgebildet.

In einer bevorzugten Ausführungsform ist der Bowdenzug einem Spannsystem zugeordnet.

In einer bevorzugten Ausführungsform kann die Seele des Bowdenzugs über ein Spannungsregelelement gespannt werden. In einer bevorzugten Ausführungsform kann die Seele des Bowdenzugs auf das Spannungsregelelement aufgerollt werden.

Dem Fachmann sind verschiedenste Spannelemente und Spannungsregelelemente wie Rollen bei medizinischen Hilfsmitteln, beispielsweise orthopädischen Hilfsmitteln, insbesondere Orthesen, oder Sporthilfsmitteln bekannt, bei denen das Spannelement, meist ein Seil oder eine Schnur, durch einen erfindungsgemäßen Bowdenzug ersetzt werden kann. Auch bei der Konstruktion neuer medizinischer Hilfsmittel oder Sporthilfsmittel kann der erfindungsgemäße Bowdenzug in vorteilhafter Weise als Spannelement eingesetzt werden.

Beim Spannen des Spannelements wird die Seele des Bowdenzugs beispielsweise durch Aufrollen oder durch Ziehen gespannt und dann fixiert. Entsprechend erfolgt in umgekehrter Weise einer Entspannung der Seele. Durch das Spannen der Seele kann beispielsweise das medizinische Hilfsmittel oder Sporthilfsmittel stärker an den Körper des Trägers angepresst werden. In vorteilhafter Weise führt die Verwendung des Bowdenzugs nun dazu, dass die Seele reibungsarm in der Ummantelung verläuft und somit nicht auf dem medizinischen Hilfsmittel oder dem Sporthilfsmittel reibt oder verklemmt. Weiterhin wird durch die Ummantelung die Auflagefläche vergrößert, sodass der Bowdenzug weniger in die Haut des Trägers einschneidet als ein herkömmliches Seil. Durch die Dehnbarkeit der Ummantelung kann sich diese in vorteilhafter Weise während des Spann- oder Entspannvorgangs an die Länge der in ihr verlaufenden Seele anpassen.

Bevorzugt umfasst das medizinische Hilfsmittel oder Sporthilfsmittel einen Grundkörper, beispielsweise aus einem Gestrick. Bevorzugt ist der Grundkörper flexibel und/oder dehnbar. Der Grundkörper kann aber auch ein Gerüst oder ähnliches sein. Bevorzugt verläuft der Bowdenzug zumindest in Teilbereichen auf dem Grundkörper.

In einer bevorzugten Ausführungsform ist die Ummantelung an ihren Endbereichen mit dem medizinischen Hilfsmittel oder Sporthilfsmittel verbunden. In einer bevorzugten Ausführungsform ist die Ummantelung nur an ihren Endbereichen mit dem medizinischen Hilfsmittel oder Sporthilfsmittel verbunden. Die Ummantelung kann alternativ oder zusätzlich auch an anderen Abschnitten oder Bereichen der Ummantelung mit dem medizinischen Hilfsmittel oder Sporthilfsmittel verbunden sein. Bevorzugt ist die Verbindung insbesondere an den Endbereichen eine feste, nicht verschiebliche Verbindung. Dagegen können auch Verbindungen vorgesehen sein, bei denen die Ummantelung verschieblich auf dem medizinischen Hilfsmittel oder Sporthilfsmittel gelagert ist, beispielsweise bei Durchführungen und/oder Umlenkführungen. Wie ein herkömmlicher Bowdenzug bei einer herkömmlichen Verwendung, kann die Ummantelung des Bowdenzugs in vorteilhafter Weise auch bei der vorliegenden Erfindung nur mit ihren Endbereichen mit der ihr zugeordneten Vorrichtung, vorliegend mit dem medizinischen Hilfsmittel oder Sporthilfsmittel, verbunden sein. Dies führt in vorteilhafter Weise dazu, dass der Längsverlauf des Bowdenzugs auf dem medizinischen Hilfsmittel oder Sporthilfsmittel verschiebbar gelagert ist und sich so beim Tragen des medizinischen Hilfsmittels oder Sporthilfsmittels an den Körperverlauf des Trägers anpassen kann und beim Spannen oder Entspannen des Spannelements auch die Ummantelung des Bowdenzugs frei auf der Fläche des medizinischen Hilfsmittels oder Sporthilfsmittels verlaufen kann, und somit nicht zu einer Faltenbildung führt.

Gemäss Erfindung sind auch Durchführungen vorgesehen, in denen die Ummantelung des Bowdenzugs verschieblich gelagert ist und die sich bevorzugt an dem medizinischen Hilfsmittel oder Sporthilfsmittel befinden, insbesondere an dem Grundkörper des medizinischen Hilfsmittels oder Sporthilfsmittels befinden. Diese Durchführungen können in vorteilhafter Weise den Bowdenzug am medizinischen Hilfsmittel oder Sporthilfsmittel positionieren ohne dass der Bowdenzug in diesem Bereich mit dem medizinischen Hilfsmittel oder Sporthilfsmittel fest verbunden ist. Die Durchführungen sind als Durchführelement ausgestaltet, beispielsweise als an dem medizinischen Hilfsmittel oder Sporthilfsmittel befestigte Röhre, durch die der Bowdenzug verläuft, insbesondere beweglich verläuft, also auch die Ummantelung verschieblich in der Röhre gelagert ist. Alternativ zu einer Röhre kann auch ein Kanal vorgesehen sein.

Die Durchführungen können auch an dem medizinischen Hilfsmittel oder Sporthilfsmittel, insbesondere an dem Grundkörper des medizinischen Hilfsmittels oder Sporthilfsmittels drehbar gelagert sein, so dass sie sich einer Verlaufsveränderung des Bowdenzugs durch entsprechende Drehung anpassen können.

Die Durchführungen können auch als Umlenkführung ausgebildet sein.

In einer bevorzugten Ausführungsform wird der Bowdenzug durch mindestens eine Umlenkführung geführt, wobei die Ummantelung in der Umlenkführung bevorzugt verschiebbar gelagert ist.

Der Bowdenzug lässt sich in vorteilhafter Weise durch Umlenkführungen führen um den Verlauf des Bowdenzugs am medizinischen Hilfsmittel oder Sporthilfsmittel festzulegen. Dabei ist der Bowdenzug in bevorzugter Weise in der Umlenkführung so gelagert, dass die Ummantelung verschiebbar ist, sodass die Umlenkführung in vorteilhafter Weise die Gesamtverschiebbarkeit der Ummantelung und des Bowdenzugs in Bezug auf das medizinische Hilfsmittel oder Sporthilfsmittel nicht beeinträchtigt.

Auch die Umlenkführungen können drehbar an dem medizinischen Hilfsmittel oder Sporthilfsmittel, insbesondere an dem Grundkörper des medizinischen Hilfsmittels oder Sporthilfsmittels drehbar gelagert sein.

In einer bevorzugten Ausführungsform wird das medizinische Hilfsmittel oder das Sporthilfsmittel am Körper getragen.

Bevorzugt handelt es sich bei dem medizinischen Hilfsmittel oder Sporthilfsmittel also nicht um solche Vorrichtungen, bei denen gewöhnlicher Weise herkömmliche Bowdenzüge verwendet werden, beispielsweise bei Bremsen von Rollatoren.

Bevorzugt handelt es sich bei dem medizinischen Hilfsmittel oder Sporthilfsmittel nicht um eine Vorrichtung, bei der Körperglieder, beispielsweise Finger durch einen Bowdenzug bewegt werden.

Bevorzugt handelt es sich bei dem medizinischen Hilfsmittel nicht um chirurgisches Hilfsmittel.

Bevorzugt handelt es sich bei dem medizinischen Hilfsmittel um ein orthopädisches Hilfsmittel.

In einer bevorzugten Ausführungsform ist das medizinische Hilfsmittel oder das Sporthilfsmittel eine Bandage oder eine Orthese.

Bevorzugt handelt es sich bei dem medizinischen Hilfsmittel oder Sporthilfsmittel nicht um eine Prothese, beispielsweise eine künstliche Hand.

Erfindungsgemäß bevorzugte Orthesen wirken äußerlich direkt am Körper. Sie dienen dazu, Gliedmaßen oder den Rumpf zu stabilisieren, zu entlasten, ruhig zu stellen, zu mobilisieren oder Fehlhaltungen zu korrigieren. Sie können auch zum Teil ausgefallene Funktionen ersetzen oder Deformationen vorbeugen. Erfindungsgemäß bevorzugt nicht mit umfasste Prothesen bilden fehlende oder geschädigte Körperteile wie Glieder, Ohren und Nase sowie Organe oder Organteile vollständig oder teilweise nach. Sie schaffen einen funktionellen Ausgleich und stellen das optische Erscheinungsbild wieder her.

Die vorliegende Erfindung löst das ihr zugrunde liegende technische Problem auch durch die Verwendung eines Bowdenzugs, aufweisend eine Seele zum Spannen eines medizinischen Hilfsmittels oder eines Sporthilfsmittels, wobei die Seele zumindest teilweise in einer Ummantelung verschiebbar verläuft und die Ummantelung dehnbar ist.

Bevorzugte Ausführungsformen des verwendeten Bowdenzugs ergeben sich aus der Beschreibung für das erfindungsgemäße medizinische Hilfsmittel oder Sporthilfsmittel umfassend einen solchen Bowdenzug.

Bevorzugte Ausführungsformen der Erfindung ergeben sich aus den Unteransprüchen.

Die vorliegende Erfindung wird an den folgenden Figuren und Beispielen näher erläutert, ohne dass diese einschränkend zu verstehen wären.

Es zeigen:
- Figur 1: Einen Bowdenzug für ein erfindungsgemäßes medizinisches Hilfsmittel oder Sporthilfsmittels;
- Figur 2: eine alternative Ausführungsformen des Bowdenzugs;
- Figur 3: den Bowdenzug als Spannsystem mit einem Spannungsregelelement;
- Figur 4: einen erfinderischen Bowdenzug mit einem Durchführungselement an einem medizinischen Hilfsmittel oder Sporthilfsmittel;
- Figur 5: Schnittdarstellung des Bowdenzugs von Figur 4;
- Figur 6: einen Bowdenzug in einer Umlenkführung;
- Figur 7: ein erfindungsgemäßes medizinisches Hilfsmittel oder Sporthilfsmittel mit einem Bowdenzug als Spannsystem;
- Figur 8: weitere alternative Ausführungsformen des Bowdenzugs.

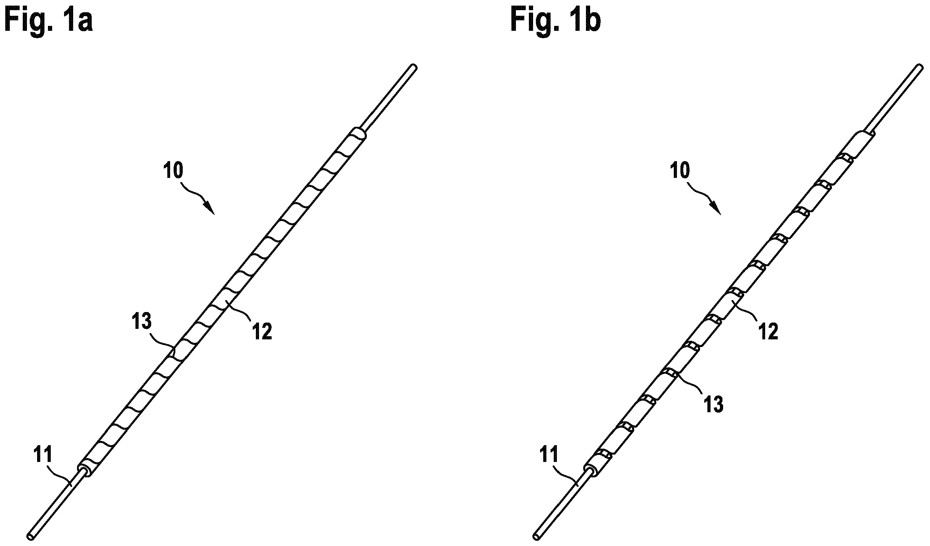

Figur 1 zeigt einen Bowdenzug (10) für ein erfindungsgemäßes medizinisches Hilfsmittel oder Sporthilfsmittel. Der Bowdenzug (10) umfasst eine Seele (11), beispielsweise aus Kunststoff oder Metall, und eine Ummantelung (12). Die Ummantelung (12) weist einen durchgehenden Schlitz (13) auf, wodurch sie dehnbar ist. Figur 1a zeigt die Ummantelung (12) im ungedehnten Zustand, Figur 1b zeigt die Ummantelung (12) in gedehntem oder gespanntem Zustand. Der Schlitz (13) ist entsprechend breiter.

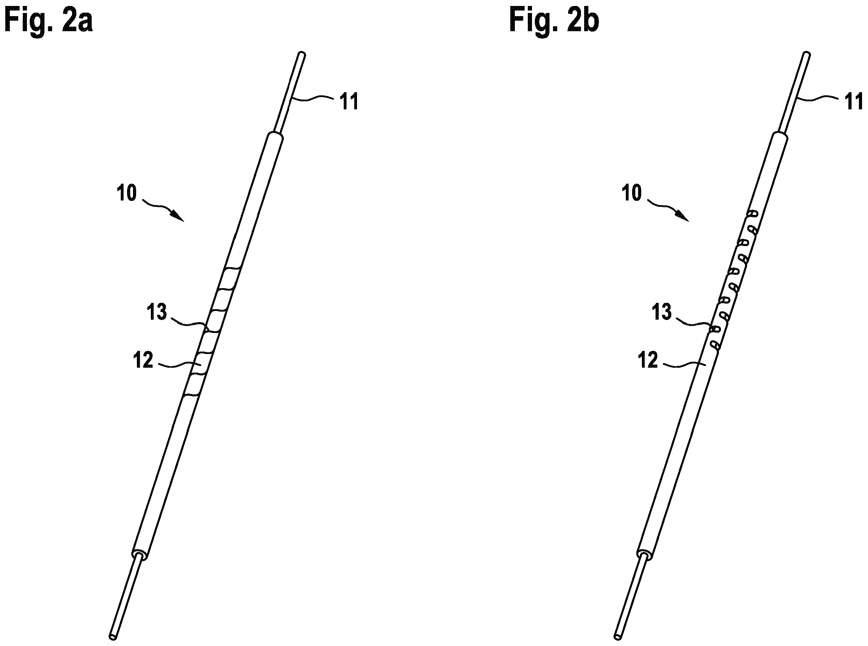

Figur 2 zeigt zwei alternative Ausführungsformen eines solchen Bowdenzugs (10). Die Seele (11) des Bowdenzugs (10) ist in den Figuren 2a und 2b gleich. In Figur 2a weist die Ummantelung (12) einen dehnbaren Bereich auf, in dem ein Schlitz (13) ringförmig oder spiralförmig verläuft. In

Figur 2b sind in dem dehnbaren Bereich der Ummantelung (12) eine Vielzahl von mäanderförmig angeordneten Schlitzen (13) vorhanden. Beide Ausführungsformen der Schlitze (13) erlauben es, dass die Ummantelung (12) im Bereich der Schlitze (13) dehnbar beziehungsweise spannbar ist.

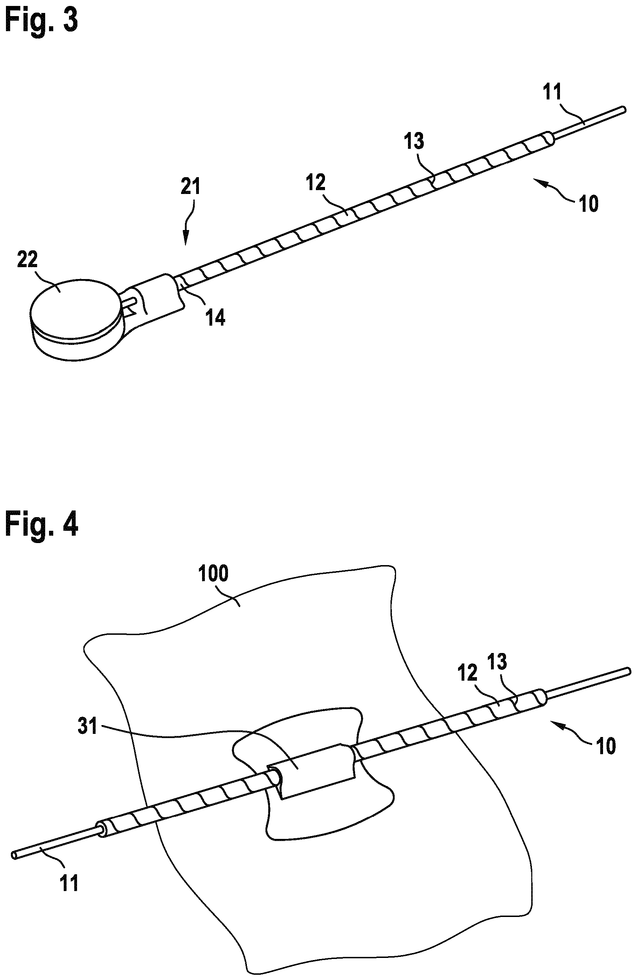

Figur 3 zeigt den Bowdenzug (10) mit Seele (11) und durch einen spiralförmigen Schlitz (13) dehnbare Ummantelung (12) die als Spannelement (21) verwendet wird. Dies wird dadurch erreicht, dass die Seele (11) auf ein Spannungsregelelement (22) aufgerollt werden kann. Beim Spannen gleitet die Seele (11) in der Ummantelung (12), wobei die Ummantelung (12) nur an ihrem Endbereich (14) mit dem Spannungsregelelement (22) verbunden ist und somit nur an diesem Endbereich (14) mit dem an Spannungsregelelement (22) befindlichen medizinischem Hilfsmittel oder Sporthilfsmittel verbunden ist. Beim Entspannen wird die Seele (11) vom Spannungsregelelement (22) abgerollt, sodass sie länger wird. Durch die Dehnbarkeit der Ummantelung (12), also durch deren spiralförmigen Schlitz (13) kann die Ummantelung sich dehnen und somit auch die verlängerte Seele (11) komplett ummanteln, sodass die Seele (11) nicht freiliegt, sondern weiterhin komplett durch die Ummantelung (12) abgeschirmt wird.

Figur 4 zeigt einen Ausschnitt eines erfindungsgemäßen medizinischen Hilfsmittels oder Sporthilfsmittels (100), dem ein Bowdenzug (10) mit Seele (11) und durch einen spiralförmigen Schlitz (13) dehnbare Ummantelung (12) zugeordnet ist. Der Bowdenzug (10) ist in dem gezeigten Bereich nicht fest an dem Trägermaterial des medizinischen Hilfsmittels oder Sporthilfsmittels (100) verbunden, sondern kann auf diesem gleiten. Dabei ist der Bowdenzug (10) durch eine als Durchführelement (31) ausgebildete röhrenförmige Durchführung verschieblich geführt. Durch das Durchführelement (31) kann nicht nur die Seele (11) des Bowdenzugs (10) gleiten, sondern auch dessen Ummantelung (12). Somit dient das Durchführelement (31) nicht der Fixierung des Bowdenzugs (10) des medizinischen Hilfsmittels oder Sporthilfsmittel (100), sondern nur zu dessen Positionierung, wobei der Bowdenzug inklusive Ummantelung in Längsrichtung weiterhin verschieblich und dehnbar bleibt.

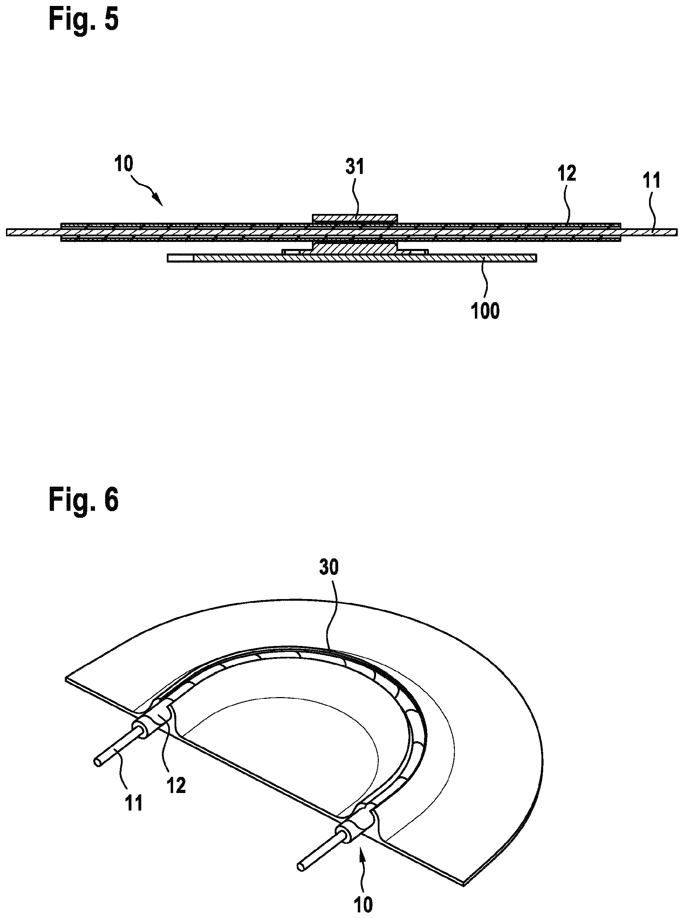

Figur 5 zeigt den Querschnitt in Seitenansicht des Aufbaus von Figur 4. Zu sehen ist der Bowdenzug (10) mit Seele (11) und dehnbarer Ummantelung (12), wobei der Bowdenzug (10) verschieblich in dem Durchführelement (31) gelagert ist, sodass eine Positionierung am Trägermaterial des medizinischen Hilfsmittels oder Sporthilfsmittels (100) erfolgt, ohne dass der Bowdenzug (10) dort fest mit dem Trägermaterial (100) verbunden ist, da der gesamte Bowdenzug (10) inklusive der Ummantelung (12) in dem Durchführelement (31) verschieblich gelagert ist.

Figur 6 zeigt einen Bowdenzug (10) in einer Durchführung, die als Umlenkelement oder Umlenkführung (30) ausgebildet ist. Auch hier ist die Ummantelung (12) des Bowdenzugs (10) verschieblich in der kanalförmigen Umlenkführung (30) gelagert, sodass zum einen der gesamte Bowdenzug (10) mit Seele (11) und Ummantelung (12) in der Umlenkführung (30) verschiebbar gelagert ist und zum anderen die Ummantelung (12) auch in der Umlenkführung (30) dehnbar bleibt. Die Umlenkführung (30) kann in einfacher Weise an ein Trägermaterial eines medizinischen Hilfsmittels oder Sporthilfsmittels befestigt werden, beispielsweise durch kleben, aufnähen oder aufschweißen.

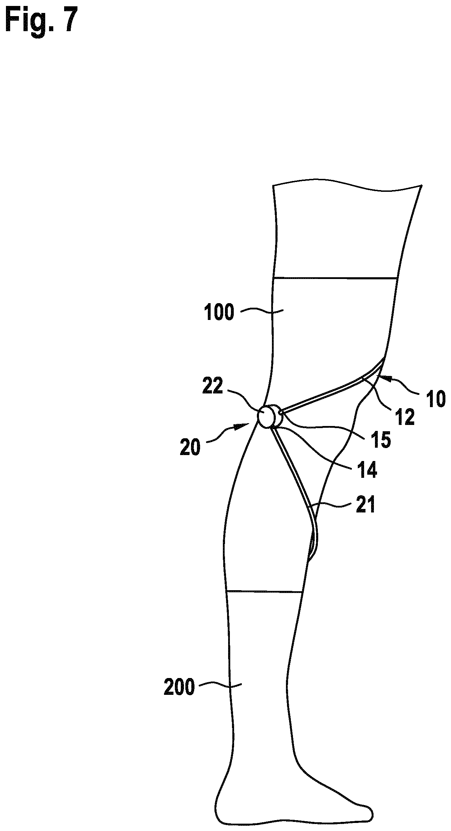

Figur 7 zeigt eine beispielhafte Ausführungsform eines medizinischen Hilfsmittels oder Sporthilfsmittels (100), das an einem Körper, hier Bein (200) getragen wird. Das medizinische Hilfsmittel oder Sporthilfsmittel (100) weist ein Spannsystem (20) auf, das einen Bowdenzug (10) mit durch Schlitzung dehnbarer Ummantelung (12) und in dieser verlaufenden, und daher nicht sichtbaren Seele als Spannelement (21) und ein Spannungsregelelement (22) umfasst. Mit dem Spannungsregelelement (22) kann die Seele des Bowdenzugs (10) aufgerollt werden und somit eine Spannung des Spannelements (21) erzeugt werden. Die dehnbare Ummantelung (12) ist dabei nur an ihren Enden (14, 15) mit dem Spannungsregelelement (22) und somit mit dem medizinischen Hilfsmittel oder Sporthilfsmittel (100) insgesamt fest verbunden. Dadurch kann sich beim Spannen oder Entspannen der Seele des Spannelements (21) die Ummantelung (12) dehnen und dabei auf dem Trägermaterial des medizinischen Hilfsmittels oder Sporthilfsmittels (100) rutschen. Dadurch ist es zum einen möglich, dass die Ummantelung (12) auch in gedehntem und gespanntem Zustand der Seele diese vollständig abdeckt, sodass die Seele nicht auf dem Trägermaterial des medizinischen Hilfsmittels oder Sporthilfsmittels (100) reibt und dieses beschädigen kann, zum anderen wird es ermöglicht, dass sich die Ummantelung (12) des Bowdenzugs (10) auf dem Trägermaterial (100) frei bewegen kann und somit eine geeignete Positionierung und einen geeigneten Verlauf des Spannelements (21) ermöglicht. Dabei ist es bei Bedarf möglich diese Positionierung durch Durchführungselemente oder Umlenkelemente, in denen der gesamte Bowdenzug (10) mit Ummantelung (12) verschiebbar gelagert ist, zu beeinflussen.

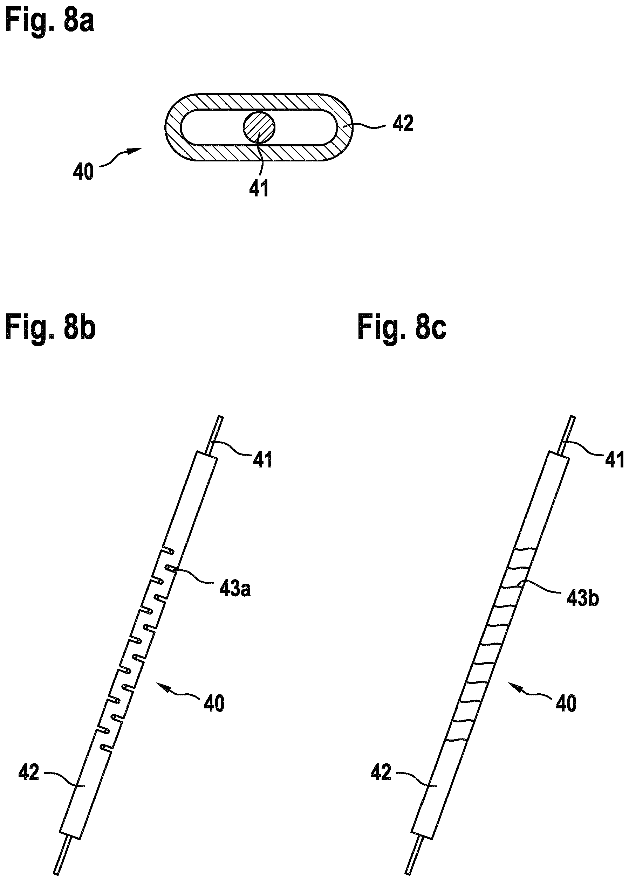

Figur 8 zeigt eine weitere Ausführungsform eines erfindungsgemäßen Bowdenzugs (40) mit einer Seele (41) und einer Ummantelung (42). In dieser Ausführungsform ist die Ummantelung (42) im Querschnitt stadionförmig ausgebildet, weist also zwei parallel verlaufende gerade Abschnitte auf, die durch zwei Bogenabschnitte miteinander verbunden sind. Durch diesen Querschnitt der Ummantelung (42) kann zum einen die Seele (41) in der Ummantelung nicht nur in Längsrichtung verschieblich gelagert sein, sondern auch in Querrichtung. Darüber hinaus kann durch diese Ausgestaltung der Ummantelung (42) als flachbandartiges Element, die Ummantelung mit einer der flachen Seiten auf dem medizinischen Hilfsmittel oder Sporthilfsmittel aufliegen. Dadurch schneidet der Bowdenzug (40) beim Anspannen nicht so sehr in die unter dem Bowdenzug (40) liegende Haut des Benutzers ein, egal ob zwischen dem Bowdenzug (40) und der Haut des Benutzers noch der Grundkörper des medizinischen Hilfsmittels oder Sporthilfsmittels liegt oder nicht.

Figur 8a zeigt den Querschnitt des Bowdenzugs (40) mit der stadionförmig beziehungsweise flachbandartig geformten Ummantelung (42) und der darin befindlichen Seele (41).

Die Figuren 8b und 8c zeigen eine Aufsicht auf einen solchen Bowdenzug (40) mit Seele (41) und Ummantelung (42), wobei die Ummantelung (42) zur Herstellung der Dehnbarkeit unterschiedliche Schlitzarten aufweist. In Figur 8b sind die Schlitze (43a) mäanderförmig ausgebildet. In Figur 8c ist ein Schlitz (43b) ringförmig, also spiralförmig ausgebildet. Sowohl in Figur 8b als auch in Figur 8c ist nur ein Teilbereich der Ummantelung (42) mit den Schlitzen (43a, 43b) versehen.

## Patentansprüche

1. Medizinisches Hilfsmittel oder Sporthilfsmittel (100), umfassend einen Bowdenzug (10), wobei der Bowdenzug (10,40) eine Seele (11,41) umfasst, wobei die Seele (11,41) zumindest in einem Teilbereich in einer Ummantelung (12,42) verläuft, wobei die Ummantelung (12,42) zumindest in einem Teilbereich dehnbar ist, wobei der Bowdenzug (10,40) als Spannelement (21) ausgebildet ist, **dadurch gekennzeichnet, dass** der Bowdenzug (10,40) durch mindestens ein Durchführelement (31) geführt wird, wobei die Ummantelung (12,42) in dem Durchführelement (31) verschiebbar gelagert ist.

2. Medizinisches Hilfsmittel oder Sporthilfsmittel (100) nach Anspruch 1, **dadurch gekennzeichnet, dass** die Ummantelung (12,42) mindestens einen Schlitz (13,43a,43b) aufweist.

3. Medizinisches Hilfsmittel oder Sporthilfsmittel (100) nach einem der vorhergehenden Ansprüche, **dadurch gekennzeichnet, dass** die Ummantelung (12,42) zumindest in einem Teilbereich spiralförmig ausgebildet ist.

4. Medizinisches Hilfsmittel oder Sporthilfsmittel (100) nach einem der vorhergehenden Ansprüche, **dadurch gekennzeichnet, dass** die Ummantelung (12,42) zumindest in einem Teilbereich elastisch ist.

5. Medizinisches Hilfsmittel oder Sporthilfsmittel (100) nach einem der vorhergehenden Ansprüche, **dadurch gekennzeichnet, dass** die Ummantelung (12,42) zumindest Abschnittsweise einen runden Querschnitt aufweist, einen eckigen Querschnitt aufweist, einen ovalen Querschnitt aufweist oder einen stadionförmigen Querschnitt aufweist.

6. Medizinisches Hilfsmittel oder Sporthilfsmittel (100) nach einem der vorhergehenden Ansprüche, **dadurch gekennzeichnet, dass** die Seele (11,41) als Draht, als Seil oder als Flachband ausgestaltet ist.

7. Medizinisches Hilfsmittel oder Sporthilfsmittel (100) nach einem der vorhergehenden Ansprüche, **dadurch gekennzeichnet, dass** der Bowdenzug (10,40) einem Spannsystem (20) zugeordnet ist.

8. Medizinisches Hilfsmittel oder Sporthilfsmittel (100) nach einem der vorhergehenden Ansprüche, **dadurch gekennzeichnet, dass** die Seele (11,41) des Bowdenzugs (10,40) über ein Spannungsregelelement (22) gespannt werden kann, insbesondere dass die Seele (11,41) des Bowdenzugs (10,40) auf das Spannungsregelelement (22) aufgerollt werden kann.

9. Medizinisches Hilfsmittel oder Sporthilfsmittel (100) nach einem der vorhergehenden Ansprüche, **dadurch gekennzeichnet, dass** die Ummantelung (12,42) an ihren Endebereichen (14,15) oder in einem oder mehreren Abschnitten mit dem medizinischen Hilfsmittel oder Sporthilfsmittel (100) verbunden ist.

10. Medizinisches Hilfsmittel oder Sporthilfsmittel (100) nach einem der vorhergehenden Ansprüche, **dadurch gekennzeichnet, dass** der Bowdenzug (10,40) durch mindestens eine Umlenkführung (30) geführt wird, wobei die Ummantelung (12,42) in der Umlenkführung (30) verschiebbar gelagert ist.

11. Medizinisches Hilfsmittel oder Sporthilfsmittel (100) nach einem der vorhergehenden Ansprüche, **dadurch gekennzeichnet, dass** das medizinische Hilfsmittel oder Sporthilfsmittel (100) einen Grundkörper aufweist.

12. Medizinisches Hilfsmittel oder Sporthilfsmittel (100) nach einem der vorhergehenden Ansprüche, **dadurch gekennzeichnet, dass** das medizinische Hilfsmittel oder das Sporthilfsmittel (100) am Körper (200) getragen wird.

13. Medizinisches Hilfsmittel oder Sporthilfsmittel (100) nach einem der vorhergehenden Ansprüche, **dadurch gekennzeichnet, dass** das medizinische Hilfsmittel oder das Sporthilfsmittel (100) eine Bandage oder eine Orthese ist.

14. Verwendung eines Bowdenzugs (10,40), aufweisend eine Seele (11,41) zum Spannen eines medizinischen Hilfsmittels oder eines Sporthilfsmittels (100), wobei die Seele (11,41) zumindest teilweise in einer Ummantelung (12,42) verschiebbar verläuft und die Ummantelung (12,42) dehnbar ist, **dadurch gekennzeichnet, dass** der Bowdenzug (10,40) durch mindestens ein Durchführelement (31) geführt wird, wobei die Ummantelung (12,42) in dem Durchführelement (31) verschiebbar gelagert ist.

## Claims

1. A medical aid or sports aid (100) comprising a Bowden cable (10), wherein the Bowden cable (10, 40) comprises a core (11, 41), wherein the core (11, 41) runs at least in a partial region in a casing (12, 42), wherein the casing (12, 42) is stretchable at least in a partial region, wherein the Bowden cable (10, 40) is designed as a tensioning element (21), **characterised in that** the Bowden cable (10, 40) is guided through at least one guide-through element (31), wherein the casing (12, 42) is displaceably mounted in the guide-through element (31).

2. The medical aid or sports aid (100) according to claim 1, **characterised in that** the casing (12, 42) has at least one slit (13, 43a, 43b).

3. The medical aid or sports aid (100) according to one of the preceding claims, **characterised in that** the casing (12, 42) is spiral-shaped at least in a partial region.

4. The medical aid or sports aid (100) according to one of the preceding claims, **characterised in that** the casing (12, 42) is elastic at least in a partial region.

5. The medical aid or sports aid (100) according to one of the preceding claims, **characterised in that** the casing (12, 42) has at least in sections a round cross-section, an angular cross-section, an oval cross-section or a stadium-shaped cross-section.

6. The medical aid or sports aid (100) according to one of the preceding claims, **characterised in that** the core (11, 41) is designed as a wire, a rope or a flat band.

7. The medical aid or sports aid (100) according to one of the preceding claims, **characterised in that** the Bowden cable (10, 40) is associated with a tensioning system (20).

8. The medical aid or sports aid (100) according to one of the preceding claims, **characterised in that** the core (11, 41) of the Bowden cable (10, 40) can be tensioned via a tension regulating element (22), in particular **in that** the core (11, 41) of the Bowden cable (10, 40) can be rolled up onto the tension regulating element (22).

9. The medical aid or sports aid (100) according to one of the preceding claims, **characterised in that** the casing (12, 42) is connected to the medical aid or sports aid (100) at its end regions (14, 15) or in one or more sections.

10. The medical aid or sports aid (100) according to one of the preceding claims, **characterised in that** the Bowden cable (10, 40) is guided through at least one deflecting guide (30), wherein the casing (12, 42) is displaceably mounted in the deflecting guide (30).

11. The medical aid or sports aid (100) according to one of the preceding claims, **characterised in that** the medical aid or sports aid (100) has a base body.

12. The medical aid or sports aid (100) according to one of the preceding claims, **characterised in that** the medical aid or sports aid (100) is worn on the body (200).

13. The medical aid or sports aid (100) according to one of the preceding claims, **characterised in that** the medical aid or sports aid (100) is a bandage or an orthosis.

14. Use of a Bowden cable (10, 40), having a core (11, 41) for tensioning a medical aid or a sports aid (100), wherein the core (11, 41) runs at least partially displaceably in a casing (12, 42) and the casing (12, 42) is stretchable, **characterised in that** the Bowden cable (10, 40) is guided through at least one guide-through element (31), wherein the casing (12, 42) is displaceably mounted in the guide-through element (31).

## Revendications

1. Accessoire médical ou accessoire de sport (100) comprenant un câble Bowden (10), dans lequel le câble Bowden (10, 40) comprend une âme (11, 41), dans lequel l'âme (11, 41) s'étend dans une gaine (12, 42) au moins dans une sous-région, dans lequel la gaine (12, 42) est extensible au moins dans une sous-région, dans lequel le câble Bowden (10, 40) est réalisé sous la forme d'un élément de mise en tension (21), **caractérisé en ce que** le câble Bowden (10, 40) est guidé à travers au moins un élément de passage (31), dans lequel la gaine (12, 42) est logée dans l'élément de passage (31) de manière à pouvoir coulisser.

2. Accessoire médical ou accessoire de sport (100) selon la revendication 1, **caractérisé en ce que** la gaine (12, 42) présente au moins une fente (13, 43a, 43b).

3. Accessoire médical ou accessoire de sport (100) selon l'une quelconque des revendications précédentes, **caractérisé en ce que** la gaine (12, 42) est réalisée en forme de spirale au moins dans une sous-région.

4. Accessoire médical ou accessoire de sport (100) selon l'une quelconque des revendications précédentes, **caractérisé en ce que** la gaine (12, 42) est élastique au moins dans une sous-région.

5. Accessoire médical ou accessoire de sport (100) selon l'une quelconque des revendications précédentes, **caractérisé en ce que** la gaine (12, 42) présente au moins par sections une section transversale ronde, présente une section transversale anguleuse, présente une section transversale ovale ou présente une section transversale en forme de stade.

6. Accessoire médical ou accessoire de sport (100) selon l'une quelconque des revendications précédentes, **caractérisé en ce que** l'âme (11, 41) est réalisée sous la forme d'un fil, d'une corde ou d'une bande plate.

7. Accessoire médical ou accessoire de sport (100) selon l'une quelconque des revendications précédentes, **caractérisé en ce que** le câble Bowden (10, 40) est associé à un système de mise en tension (20).

8. Accessoire médical ou accessoire de sport (100) selon l'une quelconque des revendications précédentes, **caractérisé en ce que** l'âme (11, 41) du câble Bowden (10, 40) peut être mise en tension par l'intermédiaire d'un élément de régulation de tension (22), en particulier **en ce que** l'âme (11, 41) du câble Bowden (10, 40) peut être enroulée sur l'élément de régulation de tension (22).

9. Accessoire médical ou accessoire de sport (100) selon l'une quelconque des revendications précédentes, **caractérisé en ce que** la gaine (12, 42) est reliée à l'accessoire médical ou à l'accessoire de sport (100) au niveau de ses régions d'extrémité (14, 15) ou dans une ou plusieurs section(s).

10. Accessoire médical ou accessoire de sport (100) selon l'une quelconque des revendications précédentes, **caractérisé en ce que** le câble Bowden (10, 40) est guidé grâce à au moins un guide de renvoi (30), dans lequel la gaine (12, 42) est logée dans le guide de renvoi (30) de manière à pouvoir coulisser.

11. Accessoire médical ou accessoire de sport (100) selon l'une quelconque des revendications précédentes, **caractérisé en ce que** l'accessoire médical ou l'accessoire de sport (100) présente un corps de base.

12. Accessoire médical ou accessoire de sport (100) selon l'une quelconque des revendications précédentes, **caractérisé en ce que** l'accessoire médical ou l'accessoire de sport (100) est porté sur le corps (200).

13. Accessoire médical ou accessoire de sport (100) selon l'une quelconque des revendications précédentes, **caractérisé en ce que** l'accessoire médical ou l'accessoire de sport (100) est un bandage ou une orthèse.

14. Utilisation d'un câble Bowden (10, 40), présentant une âme (11, 41) permettant de mettre en tension un accessoire médical ou un accessoire de sport (100), dans lequel l'âme (11, 41) s'étend au moins partiellement dans une gaine (12, 42) de manière à pouvoir coulisser et la gaine (12, 42) est extensible, **caractérisée en ce que** le câble Bowden (10, 40) est guidé à travers au moins un élément de passage (31), dans laquelle la gaine (12, 42) est logée dans l'élément de passage (31) de manière à pouvoir coulisser.
